# EUROPEAN PATENT APPLICATION

(11) **EP 2 835 055 A2**
(43) Date of publication of application: **11.02.2015**
(21) Application number: 14171327.1
(22) Date of filing: 05.06.2014
(51) Int. Cl.: A23D 9/05

(54) **Composition for polyunsaturated fatty acids encapsulation and process of preparation**

(30) Priority: 29.07.2013 US 201313953079
(71) Applicant: Corn Products Development, Inc, 04311-000SP Sao Paulo (BR)
(72) Inventor: Nik, Amir Malaki, Bedminster, NJ New Jersey 07921 (US); Li, Jason Zhixin, Martinsville, NJ New Jersey 08807 (US); Light, Joseph M., Somerset, NJ New Jersey 08873 (US); Wang, Mei Yin, 085301 Singapore (SG)
(74) Representative: Held, Stephan

(57) **Abstract**

The application relates to an encapsulated composition comprising from about 40% (w/w) to about 90% (w/w) of the encapsulating composition comprising from about 14% (w/w) to about 90% (w/w) whey protein isolate with a special ionic profile, from about 5% (w/w) to about 80% (w/w) of one or more low molecular weight carbohydrates, and from about 3% (w/w) to about 15% (w/w) antioxidant; and encapsulating from about 10% (w/w) to about 60% (w/w) polyunsaturated fatty acids.

## Description

### INTRODUCTION

Because of the health benefits of polyunsaturated fatty acids, especially, omega-3 fatty acids, there is enormous interest in fortifying foods and beverages with these substances. However, the incorporation of polyunsaturated fatty acids into foods and beverages is challenging due to the water-insolubility and highly unsaturated structures of polyunsaturated fatty acids. Because of their high unsaturation and conjugation, polyunsaturated fatty acids are particularly prone to oxidative deterioration. This limits the shelf life of foods containing polyunsaturated fatty acids because of the development of an unpleasant off-flavors and odors.

As an example of the air sensitivity of omega-3 fatty acids, linseed oil, which contains more than 50% α-linolenic acid, a specific of omega-3 fatty acid, is a drying oil. This means linseed oil will polymerize into a solid form when exposed to air. Due to its solid polymer-forming properties, linseed oil is used as a varnish in wood finishing, as a pigment binder in oil paints, as a plasticizer and hardener in putty, and in the manufacture of linoleum.

Many efforts have been made to develop effective strategies for stabilizing polyunsaturated fatty acids against lipid oxidation. Various technologies and ingredients have been utilized, yet there is no fully satisfactory solution considering performance, process, and cost. This application discloses that a whey protein isolate with a special ionic profile, combined with low molecular weight carbohydrates, provides a material with superior encapsulation performance and which is cost-effective. IN addition, the material protects the encapsulated polyunsaturated fatty acids against oxygen.

### SUMMARY

In one aspect the application provides an encapsulated composition comprising from about 40% (w/w) to about 90% (w/w) of the encapsulating composition comprising from about 14% (w/w) to about 90% (w/w) whey protein isolate with a special ionic profile, from about 5% (w/w) to about 80% (w/w) of one or more low molecular weight carbohydrates, and from about 3% (w/w) to about 15% (w/w) antioxidant; and encapsulating from about 10% (w/w) to about 60% (w/w) polyunsaturated fatty acids.

### BRIEF DESCRIPTION OF THE DRAWING

Fig. 1 depicts an encapsulation process for polyunsaturated fatty acids.
Fig. 2 depicts the results of oxidation of polyunsaturated fatty acids encapsulated with the whey protein isolate with a special ionic profile (WPI-SI) and commercial whey protein isolates (WPI1, WPI2, WPI3, WPI4, WPI5) as measured by head-space GC.
Fig. 3 depicts the sensory perception evaluation of polyunsaturated fatty acids encapsulated with the whey protein isolate with a special ionic profile (WPI-SI) and commercial whey protein isolates (WPI1, WPI2, WPI3, WPI4, WPI5).

### DETAILED DESCRIPTION

In one aspect the application provides an encapsulated composition comprising from about 40% (w/w) to about 90% (w/w) of the encapsulating composition comprising from about 14% (w/w) to about 90% (w/w) whey protein isolate with a special ionic profile, from about 5% (w/w) to about 80% (w/w) of one or more low molecular weight carbohydrates, and from about 3% (w/w) to about 15% (w/w) antioxidant; and encapsulating from about 10% (w/w) to about 60% (w/w) polyunsaturated fatty acids.

In one embodiment the application provides the encapsulated composition wherein the encapsulating composition comprises from about 16% (w/w) to about 75% (w/w) whey protein isolate with a special ionic profile and from about 21% (w/w) to about 76% (w/w) of one or more low molecular weight carbohydrates.

In another embodiment the application provides the encapsulated composition wherein the encapsulating composition comprises from about 18% (w/w) to about 58% (w/w) whey protein isolate with a special ionic profile and from about 37% (w/w) to about 74% (w/w) of one or more low molecular weight carbohydrates.

In another embodiment the application provides the encapsulated composition wherein the encapsulating composition comprises from about 20% (w/w) to about 42% (w/w) whey protein isolate with a special ionic profile and from about 53% (w/w) to about 72% (w/w) of one or more low molecular weight carbohydrates.

In another embodiment the application provides the encapsulated composition wherein the encapsulating composition comprises from about 21% (w/w) to about 27% (w/w) whey protein isolate with a special ionic profile and from about 68% (w/w) to about 72% (w/w) of one or more low molecular weight carbohydrates.

In another embodiment the application provides the encapsulated composition wherein the encapsulating composition comprises about 23% (w/w) whey protein isolate with a special ionic profile and about 70% (w/w) of one or more low molecular weight carbohydrates.

In one embodiment the application provides the encapsulated composition wherein the one or more low molecular weight carbohydrates is a mixture of sucrose and corn syrup solids.

In another embodiment the application provides the encapsulated composition wherein the mixture of sucrose and corn syrup solids is a mixture from about 33% (w/w) to about 67% (w/w) sucrose and from about 67% (w/w) to about 33% (w/w) corn syrup solids.

In another embodiment the application provides the encapsulated composition wherein the mixture of sucrose and corn syrup solids is a mixture of about 50% (w/w) sucrose and of about 50% (w/w) corn syrup solids.

In another embodiment the application provides the encapsulated composition wherein the encapsulating composition comprises from about 4% (w/w) to about 10% (w/w) antioxidant.

In another embodiment the application provides the encapsulated composition wherein the encapsulating composition comprises from about 6% (w/w) to about 8% (w/w) antioxidant.

In another embodiment the application provides the encapsulated composition wherein the antioxidant is ascorbic acid.

In another embodiment the application provides the encapsulated composition wherein the polyunsaturated fatty acids are omega-3 fatty acids.

In one embodiment the application provides the method of making the encapsulated composition comprising from about 40% (w/w) to about 90% (w/w) of the encapsulating composition comprising from about 14% (w/w) to about 90% (w/w) whey protein isolate with a special ionic profile, from about 5% (w/w) to about 80% (w/w) of one or more low molecular weight carbohydrates, and from about 3% (w/w) to about 15% (w/w) antioxidant; and encapsulating from about 10% (w/w) to about 60% (w/w) polyunsaturated fatty acids comprising encapsulating the ingredients in water and drying.

In another embodiment the application provides the method of making the encapsulated composition wherein the drying is spray drying.

The encapsulated composition of this application comprises whey protein isolate with a special ionic profile (WPI-SI) and low-molecular weight carbohydrates. In one example, at a ratio of 1:3 (w/w), the encapsulated composition of this application provides excellent oxidative protection especially at high oil load (40%). The low molecular weight carbohydrate part can be selected from but not limited to maltodextrin, corn syrup solids, lactose, sucrose, or their combinations. Optionally, ascorbic acid (as an antioxidant) can be included into the composition. The components of the invention and their blend ratios are selected based on their ability to form stable emulsion and create effective wall material for omega-3 fatty acid protection from oxidation.

The application relates to an encapsulation composition for polyunsaturated fatty acids (PUFA) comprising a whey protein isolate with special ionic profile, known as heat-stable whey protein isolate (WPI-SI), and low-molecular weight carbohydrates. The application also provides a method for preparation of omega-3 fatty acids microcapsules. The application enables 40% oil load encapsulation of omega-3 fatty acids, and provides excellent oxidative protection, thus a great sensory impression.

The polyunsaturated fatty acids may be encapsulated using the whey protein isolate with special ionic profile and low-molecular weight carbohydrates encapsulating composition of the present application and techniques known in the art. In one embodiment, the whey protein isolate with special ionic profile and low-molecular weight carbohydrates encapsulating composition may be dispersed in water, the polyunsaturated fatty acids may be added and emulsified, and the emulsion may then be dried to form the encapsulated material. Drying may be accomplished by any appropriate method known in the art, including but not limited to spray drying, extrusion, spray chilling, and fluid bed coating. In one embodiment, the polyunsaturated fatty acids are homogenized (emulsified) in a solution/dispersion of the whey protein isolate with special ionic profile and low-molecular weight carbohydrates encapsulating composition and then spray dried. Emulsification and drying conditions may be controlled by one skilled in the art to yield encapsulated material with the desired attributes. For example, if volatile or heat labile polyunsaturated fatty acids are used, relatively low temperatures will be used to reduce loss and/or inactivation of the polyunsaturated fatty acids. One skilled in the art may also vary the average particle size of the emulsion to obtain the desired results. In one embodiment, the average particle size of the emulsion is from about 0.1 micron to about two micron.

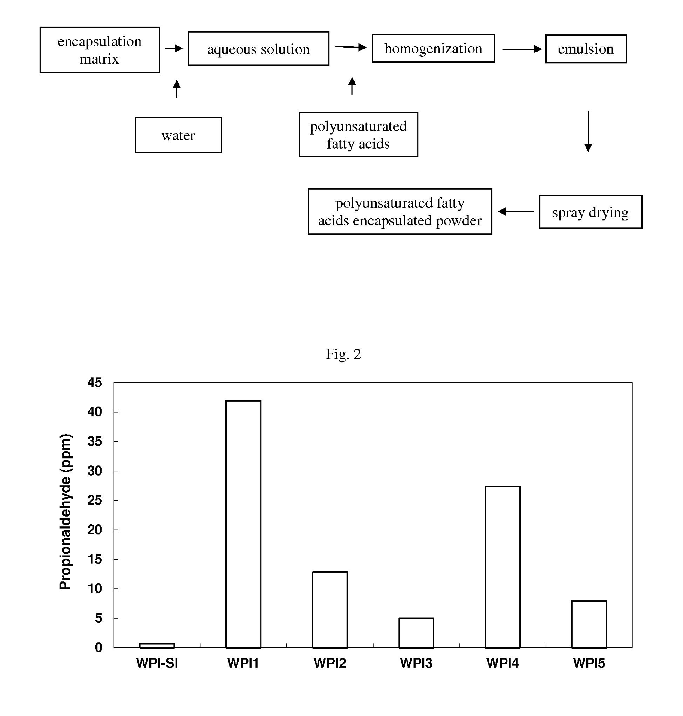

Fig. 1 is a flowchart of the polyunsaturated fatty acids encapsulation process illustrating the steps. The process starts with dispersing the encapsulating composition of the application in water followed by pH adjustment to a value of 3 using citric, hydrochloric, or phosphoric acid (if necessary). The process continues with emulsification of polyunsaturated fatty acids in aqueous solution of the matrix materials using an APV pressure homogenizer at 5000 psi for two passes. Stable emulsions with mean droplet diameter of 1-2 µm were recovered after the homogenization step. Using spray drying technology, the polyunsaturated fatty acids containing emulsion was transformed to free flowing powder. The spray drying process was conducted with inlet temperature of about 160 °C and outlet temperature of about 90 °C.

The encapsulated material prepared with the present encapsulating agents consistently achieves and maintain a relatively high level of the polyunsaturated fatty acids. The polyunsaturated fatty acids may be present in an amount of from about 10%(w/w) to about 60% (w/w) based upon the encapsulated material (the whey protein isolate with special ionic profile and low-molecular weight carbohydrates encapsulating composition plus polyunsaturated fatty acids). In another embodiment, the active agent is present in an amount of from about 25% (w/w) to about 40% (w/w).

The resultant encapsulated material may be used in various food products including, but not limited to, infant formula, cereals; powdered drink mixes; instant coffees and teas; powdered sauce and gravy mixes; instant soups; powdered dressings; bakery products including breads and bread products; intermediate moisture foods including shelf stable nutrition bars; flavors; fragrances; colorants; and other dry food products. Upon preparation of powdered and instant products, the moisture triggers the release mechanism, providing the polyunsaturated fatty acids to the consumer.

### DEFINITIONS

The following acronyms, definitions, and abbreviations are used in connection with the processes of the present application unless the context indicates otherwise. The abbreviation "(w/w)", as used herein, means the true percentage by weight. That is, the weight of the ingredient is divided by the total weight in the same units of the composition. The abbreviation "% (w/w)" means the weight of the ingredient divided by the total weight of the composition and converted to a percentage. The acronym ALA means α-linolenic acid. The acronym EPA means eicosapentaenoic acid. The acronym DHA means docosahexaenoic acid. The acronym PUFA means polyunsaturated fatty acid. The acronym "DE" means Dextrose Equivalent. "DE" is defined as the reducing power of starch derivatives and sugars. Each starch molecule has one reducing end; therefore DE is inversely related to molecular weight. The DE of anhydrous D-glucose is defined as 100 and the DE of unhydrolyzed starch is virtually zero. The acronym "ppm" means parts per million and is equivalent to a concentration expressed as mg/L.

As used herein, the word, "antioxidant" refers to compounds that prevent oxidative degradation of foods that effect odor and/or flavor. "Antioxidants" include, but are not limited to L-ascorbic acid, sodium ascorbate, calcium ascorbate, potassium ascorbate, ascorbyl stearate, ascorbyl palmate, α-tocopherol, citric acid, erthorbic acid, propyl gallate, butylated hydroxyl anisole (BHA), butylated hydroxyl toluene (BHT), tertiary butyl hydroquinone (TBHQ), 2,6-ditertiary-butyl-4-hydroxy-methylphenol (ionox-100), and 2,4,6-trihydroxy butylphenone (THBP).

As used herein, the word, "corn syrup solids" refers to the dry form of corn syrup, a sweetener derived from corn. "Corn syrup solids" are less sweet than sucrose and are available in three different sweetness levels or dextrose equivalent (DE) ratings.

As used herein, the phrase, "low molecular weight carbohydrate" refers to monosaccharaides, disaccharides, oligosaccharides, corn syrup solids, and maltodextrins. The low molecular weight carbohydrate suitable for the present application include, but are not limited to, debranched starches, sucrose, dextrose, lactose, fructose, maltose, or corn syrup solids with a DE of from about 20 to 60. The source of the low molecular weight carbohydrate may be any native source such cereals, tubers, roots, legumes and fruits. The native source can be corn, pea, potato, sweet potato, banana, barley, wheat, rice, sago, amaranth, tapioca, arrowroot, canna, sorghum, and waxy or high amylose varieties thereof.

As used herein, the phrase "omega-3 fatty acids" refers to three compounds α-linolenic acid (found in plant oils), eicosapentaenoic acid, and docosahexaenoic acid (both commonly found in marine oils). Common animal sources of eicosapentaenoic acid and docosahexaenoic acid include fish oils, algal oil, egg oil, squid oils, and krill oil. Some plant oils such as seabuckthorn seed and berry oils, flaxseed oil, Sacha Inchi oil, Echium oil, and hemp oil contain α-linolenic acid. Omega-3 fatty acids are vital for normal metabolism. Omega-3 fatty acids are considered essential fatty acids since they cannot be normally synthesized by the human body. Mammals have a limited ability, when the diet includes the shorter-chained C-18 α-linolenic acid, to make the more important longer-chained C-20 eicosapentaenoic acid and then to make the most crucial lonest chain C-22 docosahexaenoic acid with even less efficiency.

As used herein, the phrase "polyunsaturated fatty acids" refers to carboxylic acids with a long aliphatic chain, which contain more than one double bond between carbon atoms in the chain. They contain from six to 28 carbon atoms including the carboxyl group. "Polyunsaturated fatty acids" include the essential fatty acids, omega fatty acids, and linoleic acid. Polyunsaturated fatty acids are found in certain foods and can benefit health by lowering cholesterol levels and providing essential lipids known as omega-3 fatty acids and omega-6 fatty acids. Polyunsaturated fatty acids can be found in seafood, sunflower-seed, corn, sesame, soy and safflower oil peanut, walnuts, and grains.

As used herein, the phrase "whey protein isolate" refers to a composition made by filtering whey, a by-product of the cheese-making process. Whey protein isolate has a higher percentage of pure protein than do other forms of whey protein. "Whey protein isolate" is virtually fat free, cholesterol, free and at least 80% protein by weight. Two filtration methods are widely used commercially to make "whey protein isolate". One method, utilizes ion exchangers and the other method, microfiltration, uses passage through a microporous membrane.

As used herein, the phrase "whey protein isolate with a special ionic profile" refers to whey protein isolates containing less than 75 ppm sodium, less than 45 ppm potassium, less than 300 ppm calcium, and more than 7,000 ppm phosphorus.

Certain specific aspects and embodiments of the present application are explained in more detail with reference to the following embodiments and examples, which are provided only for purposes of illustration and should not be construed as limiting the scope of the application in any manner.

### EMBODIMENTS

1. An encapsulated composition comprising:
   a) from about 40% (w/w) to about 90% (w/w) of the encapsulating composition comprising:
      i) from about 14% (w/w) to about 90% (w/w) whey protein isolate with a special ionic profile,
      ii) from about 5% (w/w) to about 80% (w/w) of one or more low molecular weight carbohydrates, and
      iii) from about 3% (w/w) to about 15% (w/w) antioxidant; and
   b) encapsulating from about 10% (w/w) to about 60% (w/w) polyunsaturated fatty acids.
2. The encapsulated composition of embodiment 1 wherein the encapsulating composition comprises from about 16% (w/w) to about 75% (w/w) whey protein isolate with a special ionic profile and from about 21% (w/w) to about 76% (w/w) of one or more low molecular weight carbohydrates.
3. The encapsulated composition of embodiment 2 wherein the encapsulating composition comprises from about 18% (w/w) to about 58% (w/w) whey protein isolate with a special ionic profile and from about 37% (w/w) to about 74% (w/w) of one or more low molecular weight carbohydrates.
4. The encapsulated composition of embodiment 3 wherein the encapsulating composition comprises from about 20% (w/w) to about 42% (w/w) whey protein isolate with a special ionic profile and from about 53% (w/w) to about 72% (w/w) of one or more low molecular weight carbohydrates.
5. The encapsulated composition of embodiment 4 wherein the encapsulating composition comprises about 23% (w/w) whey protein isolate with a special ionic profile and about 70% (w/w) of one or more low molecular weight carbohydrates.
6. The encapsulated composition of embodiment 1 wherein the one or more low molecular weight carbohydrates is a mixture of sucrose and corn syrup solids.
7. The encapsulated composition of embodiment 6 wherein the mixture of sucrose and corn syrup solids is a mixture of about 50% (w/w) sucrose and of about 50% (w/w) corn syrup solids.
8. The encapsulated composition of embodiment 1 wherein the encapsulating composition comprises from about 4% (w/w) to about 10% (w/w) antioxidant.
9. The encapsulated composition of embodiment 8 wherein the encapsulating composition comprises from about 6% (w/w) to about 8% (w/w) antioxidant.
10. The encapsulated composition of embodiment 1 wherein the antioxidant is ascorbic acid
11. The encapsulated composition of embodiment 1 wherein the polyunsaturated fatty acids are omega-3 fatty acids.
12. A method of making the encapsulated composition of embodiment 1 comprising emulsifying the ingredients in water and drying.
13. The method of embodiment 12 wherein the drying is spray drying.
14. The method of embodiment 12 wherein the antioxidant is ascorbic acid.
15. The method of embodiment 12 wherein the polyunsaturated fatty acids are omega-3 fatty acids.

### EXAMPLES

**Head space GC analysis procedure.** Samples were analyzed to determine the degree of oxidation of C3 aldehyde by HS GC using a six-point calibration curve for propionaldehyde. A Hewlett-Packard 7890A GC/FID system was used for the analysis. Column 30 m. x 0.53 mm ID Stabilwax-DA (1.5 µm film), helium carrier 20 mL/minute constant flow, oven 50 °C for 2.5 minutes; 15 °C/min. to 200 °C; hold 2.5 minutes (15 min. run), injection port temperature 200 °C, detector temperature 250 °C. A G1888 Network Headspace Sampler was used in the analysis. Oven temperature 80 °C, loop temperature 80 °C, transfer line temperature 90 °C, vial equilibrium time 20 minutes, pressurization time 0.2 minute, injection time 1 minute, and GC cycle 15 minutes. The results are given in units of µg/g (PPM) unless otherwise noted.

**Measurement of the emulsion droplet size procedure.** The average size of the emulsion particles was measured using Beckman Coulter Laser Diffraction Particle Size Analyzer.

**Sensory evaluation procedure.** The omega-3 encapsulated powders were mixed with infant milk follow in the amount of 100 mg/100g of the infant formula. A 14 g portion of the above mixture were reconstituted with 60 °C water, cooled to 40 °C, and served. Test samples were evaluated at 30 °C to 40 °C. The samples were ranked based on their fishy note from 0 to 5, where 0 had the least fishy note and 5 had the fishiest note. Three evaluators scored the samples and their scores were averaged.

**Mineral analysis procedure.** The samples were digested by microwave digestion. Sample sizes of approximately 200 mg (weighed to the nearest 0.1mg) were digested in 5 mL nitric acid and 3 mL 30% hydrogen peroxide using the following profile: ramping to 150 °C over 10 minutes and holding for 5 minutes, then ramping to 260 °C over 10 minutes and holding for 10 minutes. After cooling to room temperature, the samples were gravimetrically diluted (approximately 20 g final weight) and internal standard was added prior to analysis by ICP. Quantification was achieved using a four-level calibration with linear regression. Duplicate blanks were prepared and analyzed in parallel with the samples and subtracted from the sample results. The analytes of interest were sodium, potassium, calcium, sulfur, phosphorus, magnesium, and iron.

**Example 1 encapsulation composition preparation procedure.** A mixture of 233.3 g whey protein isolate with a special ionic profile, 350 g corn syrup solids 42DE, 350 g sucrose, and 66.6 g ascorbic acid was dissolved in 1500 mL DI water. The solution was spray dried using a Niro Utility Spray Drier # 3-068 with a centrifugal atomizer installed.

| ingredient | % (w/w) |
|---|---|
| whey protein isolate with a special ionic profile | 23.33 |
| corn syrup solids | 35 |
| sucrose | 35 |
| ascorbic acid | 6.66 |
| total | 100 |

**Example 2 encapsulation composition preparation procedure.** A mixture of 233.3 g whey protein isolate with a special ionic profile, 700 g corn syrup solids 42DE, and 66.6 g ascorbic acid was dissolved in 1500 mL DI water. The solution was spray dried using a Niro Utility Spray Drier # 3-068 with a centrifugal atomizer installed.

**Example 3 Encapsulation Process.** Fig. 1 is a flowchart of the omega-3 fatty acids encapsulation process illustrating the steps. The process started with dispersing the 360 g of encapsulating composition of Example 1, as described above, in water followed by pH adjustment to a value of around 3 using citric acid. A 250 g portion of omega-3 oil was added into the aqueous solution. The mixture was pre-homogenized using a high shear mixture at 5000 rpm for 2 minutes. This pre-emulsion was then homogenized using an APV homogenizer at 5000 psi for two passes to reach an average particle size of approximately 1 micron. The emulsion was spray-dried using a Niro Utility Spray Drier # 3-068 with a centrifugal atomizer installed. The inlet temperature was approximately 160 °C and the outlet temperature approximately 90 °C. The flow rate was kept at about 50 mL/min.

| Ingredient | % (w/w) | % (w/w) |
|---|---|---|
| whey protein isolate with a special ionic profile | 18 | 14 |
| corn syrup solids | 26.5 | 21 |
| Sucrose | 26.5 | 21 |
| ascorbic acid | 4 | 4 |
| Omega-3 fatty acids | 25 | 40 |
| Total | 100 | 100 |

**Example 4.** The ability of the new encapsulation compositions to protect omega-3 fatty acids was evaluated by measuring the amount of propionaldehyde, a major product of omega-3 fatty acid oxidation, using head-space gas chromatography after aging the powder at 63 °C for 12 days in combination with sensory evaluation. The ability of compositions of the application to protect omega-3 fatty acids was evaluated compare to various type of whey protein isolates (*i.e.* native, heat-stable, and hydrolyzed) and sodium caseinate in combination with above mentioned carbohydrates. A mixture of corn syrup solids and sucrose in a 50:50 (w/w) ratio was used. As shown in Fig 2. the encapsulated composition of the application outperformed other type of the proteins for omega-3 fatty acid protection. In other words, below 1 ppm lipid oxidation was observed during 12 days storage at 63 °C.

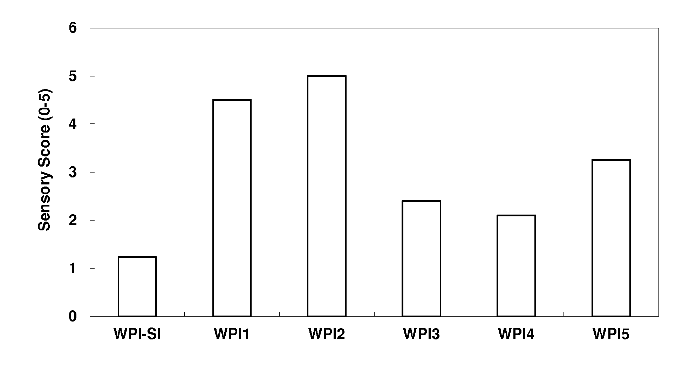

**Example 5.** The omega-3 fatty acids microcapsule was also evaluated for sensory perception. For this purpose, the aged omega-3 fatty acids encapsulated powders were mixed with non-fat milk powder and samples were scored based on perception of fishy smell and taste using the procedure described above. The results are shown in Fig. 3. Overall, the stronger smell and taste was observed from prototypes with higher level of oxidation. Among all the prototypes, the prototype made from application composition showed least fishy note due to significantly lower omega-3 fatty acids oxidation.

**Example 6.** The superior performance of the whey protein isolate with a special ionic profile used in this application for omega-3 fatty acids encapsulation can be associated with its unique mineral contents. Minerals content analysis revealed that the Whey protein isolate with a special ionic profile used in this application contains significantly lower amount of calcium, potassium, and sodium compared to native whey protein isolate, while the phosphate content was found significantly higher as seen in Table 1. High phosphate content contributes strongly to the performance of the application. Without being bound by theory either through improving the antioxidant properties of the encapsulating material perhaps by affecting the protein structure and functionality.

**Table 1 The mineral content of whey protein isolate with a special ionic profile and native-whey protein isolate**

| sample | Na content | K content | Ca content | P content |
|---|---|---|---|---|
| whey protein isolate with a special ionic profile WPI-SI | 10 | 22 | 148 | 15,000 |
| commercial-whey protein isolate WPI1 | 3,888 | 1,294 | 4284 | 1799 |
| commercial-whey protein isolate WPI2 | 7,101 | 235 | 619 | 612 |
| commercial-whey protein isolate WPI3 | 137 | 16 | 37 | 1597 |
| commercial-whey protein isolate WPI4 | 595 | 20 | 649 | 341 |
| commercial-whey protein isolate WPI5 | 1,699 | 2,819 | 5,166 | 25,700 |

The concentrations are in ppm.

Throughout this application, various publications are referenced. The disclosures of these publications in their entireties are hereby incorporated by reference into this application in order to more fully describe the state of the art as known to those skilled therein as of the date of the application described and claimed herein. While particular embodiments of the present application have been illustrated and described, it would be obvious to those skilled in the art that various other changes and modifications can be made without departing from the spirit and scope of the application. It is therefore intended to cover in the appended claims all such changes and modifications that are within the scope of this application.

## Claims

1. An encapsulated composition comprising:
a) from about 40% (w/w) to about 90% (w/w) of the encapsulating composition comprising:
i) from about 14% (w/w) to about 90% (w/w) whey protein isolate with a special ionic profile,
ii) from about 5% (w/w) to about 80% (w/w) of one or more low molecular weight carbohydrates, and
iii) from about 3% (w/w) to about 15% (w/w) antioxidant; and
b) encapsulating from about 10% (w/w) to about 60% (w/w) polyunsaturated fatty acids.

2. The encapsulated composition of claim 1, wherein the encapsulating composition comprises from about 20% (w/w) to about 42% (w/w) whey protein isolate with a special ionic profile and from about 53% (w/w) to about 72% (w/w) of one or more low molecular weight carbohydrates.

3. The encapsulated composition of claim 1 or 2, wherein the encapsulating composition comprises about 23% (w/w) whey protein isolate with a special ionic profile and about 70% (w/w) of one or more low molecular weight carbohydrates.

4. The encapsulated composition of any one of claims 1-3, wherein the one or more low molecular weight carbohydrates is a mixture of sucrose and corn syrup solids.

5. The encapsulated composition claim 4, wherein the one or more low molecular weight carbohydrates is a mixture of about 50% (w/w) sucrose and of about 50% (w/w) corn syrup solids.

6. The encapsulated composition of any one of claims 1-5, wherein the encapsulating composition comprises from about 4% (w/w) to about 10% (w/w) antioxidant.

7. The encapsulated composition of any one of claims 1-6, wherein the antioxidant is ascorbic acid

8. The encapsulated composition of any one of claims 1-7, wherein the polyunsaturated fatty acids are omega-3 fatty acids.

9. A method of making the encapsulated composition of any one of claims 1-8, comprising emulsifying the ingredients in water and drying.

10. The method of claim 9, wherein the drying is spray drying.
